(19)

Europäisches Patentamt
European Patent Office
Office européen des brevets

(11) **EP 4 332 150 A1**

(12) **EUROPEAN PATENT APPLICATION**

(43) Date of publication:
06.03.2024 Bulletin 2024/10

(21) Application number: 22193846.7

(22) Date of filing: 05.09.2022

(51) International Patent Classification (IPC):
**C08J 3/00** $^{(2006.01)}$     **G01N 33/44** $^{(2006.01)}$
**C08J 11/06** $^{(2006.01)}$

(52) Cooperative Patent Classification (CPC):
**C08J 3/005; C08J 11/06;** C08J 2300/30;
C08J 2323/06; C08J 2400/30; C08J 2423/06;
G01N 33/442

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Borealis AG**
**1020 Vienna (AT)**

(72) Inventors:
• **LOPES FILIPE, Susana**
**7005-545 Évora (PT)**

• **NAGL, Andreas**
**4021 Linz (AT)**
• **TRAN, Tuan Anh**
**4021 Linz (AT)**
• **MACHL, Doris**
**4021 Linz (AT)**
• **GOSWAMI, Mithun**
**4021 Linz (AT)**
• **LIU, Yi**
**4021 Linz (AT)**

(74) Representative: **Maiwald GmbH**
**Engineering**
**Elisenhof**
**Elisenstrasse 3**
**80335 München (DE)**

(54) **METHOD OF BLENDING POLYETHYLENE BASED BLENDS**

(57)    The present invention relates to a method of blending polyethylene based blends based on a correlation between the at least one first property related to the broadness of the molecular weight distribution curve of polyethylene based blends, the at least one second property related to the molecular weight of polyethylene based blends and the one property related to the target property of a certain application for which the blended polyethylene based blends are intended to be used, polyethylene blends blended by said method having a time to failure of at least 5 hours in full notch creep test (FNCT), determined according to ISO 16770 or a time to failure of at least 10 hours in environmental stress crack resistance (ESCR) test of bottle according to ASTM D2561, and articles comprising said polyethylene blends.

Fig. 1

EP 4 332 150 A1

## Description

The present invention relates to a method of blending polyethylene based blends based on a correlation between the at least one first property related to the broadness of the molecular weight distribution curve of polyethylene based blends, the at least one second property related to the molecular weight of polyethylene based blends and the one property related to the target property of a certain application for which the blended polyethylene based blends are intended to be used, polyethylene blends blended by said method having a time to failure of at least 5 hours in full notch creep test (FNCT), determined according to ISO 16770 or a time to failure of at least 10 hours in environmental stress crack resistance (ESCR) test of bottle according to ASTM D2561, and articles comprising said polyethylene blends.

## Technical background

Polyethylene resins such as high density polyethylene (HDPE) resins, are used for demanding high end applications, such as pipes and pipe fittings prepared by extrusion, injection moulding or containers and bottles prepared by blow moulding or automotive applications. For such high end applications mechanical and thermal long term stability are key properties. Standard tests for long term stability such as slow crack growth (SCG) resistance are e.g. environmental stress crack resistance (ESCR) which can be measured according to ASTM D2561 or full notch creep test (FNCT), which can be measured according to ISO 16770. SCG resistance generally depends on molecular weight, molecular weight distribution, comonomer content and comonomer distribution of the polyethylene resin.

Polyethylene resins meeting high end demands can either be produced by in situ polymerization in elaborate multistage polymerization processes, in which the partly contradicting properties are reached by polymerizing different polyethylene components in different reaction stages or by mechanically blending polyethylene resins.

Mechanical blends allow the production of high end blends from rather cheap starting materials but have the drawback that due to variances in the starting material the end properties for each blend needs to be verified again. Especially for the long term properties like ESCR or FNCT the tests can run over several months.

Suitable starting materials are virgin polyethylene resins, especially cheap virgin polyethylene resins produced in a single reaction stage, and recycled polyethylene blends obtained from sorting processes for sorting post-consumer waste and/or post-industrial waste.

Especially blending recycled polyethylene blends faces several challenges:
Most commercial HDPE grades are produced with Ziegler-Natta or Phillips Chromium catalysts; whereas some HDPE grades are produced with single-site catalysts. Hence the mechanical properties of the final product are shaped by combination of all these factors.

On the other hand, mechanical properties of recycled polyethylenes including ESCR, are influenced not only by polymer structure but by contaminants. This adds uncertainties to mechanical performance of recycled polyethylene.

As a consequence recycled polyethylene blends vary in their properties in each batch depending on their feedstock even if the same sorting process is used on different batches. This means that for using recycled polyethylene blends in high end applications it is not clear whether the high end properties are met by each and every batch.

Therefore, when using mechanical blends, especially including or consisting of recycled polyethylene blends, in high end applications for each batch it is necessary to determine the high end properties including the long term properties, which is cost intensive and time consuming.

Thus, there is a need in the art for establishing correlations, which allow predicting high end properties of polyethylene based blends based on structural properties, which preferably are routinely measured or at least can be easily measured without high effort.

The present invention is based on the surprising finding of correlations of target properties for high end applications with properties related to the broadness of the molecular weight distribution curve and to the molecular weight of polyethylene based blends, which are easily accessible. This allows to predict target properties, especially long term properties for polyethylene based blends such as ESCR or FCNT, which measurements usually take up to several months from easily accessible measurements, which can easily be conducted or already are routine measurements for polyethylene resins. Such correlations are especially suitable for recycled polyethylene blends, which can significantly vary in their properties in each charge, so that for high end uses technically for each charge the high end properties need to be determined. A suitable correlation allows for a fairly accurate prediction of the accordant high end property needed for an application for which the material is intended to be used, helps saving time and costs and helps incorporation of higher amounts of recycled polyethylene blends also in high end applications.

## Summary of the invention

The present invention relates to a method of blending polyethylene based blends comprising the steps of

- Providing a first amount of polyethylene based blends;
- Measuring properties of said first amount of polyethylene based blends, wherein at least one first property is related to the broadness of the molecular weight distribution curve of each of the first amount of polyethylene based blends, at least one second property is related to the molecular weight of each of the first amount of polyethylene based blends and one property related to the target property of a certain application for which the blended polyethylene based blends are intended to be used;
- Establishing a correlation between the at least one first property, the at least one second property and the one property related to the target property;
- Providing a second amount of polyethylene based blends;
- Measuring the same at least one first property and at least one second property, that were measured of the first amount of polyethylene based blends, of each of the second amount of polyethylene based blends;
- Using the correlation between the first property, the second property and the property related to the target property established for the first amount of polyethylene based blends for predicting the one property related to the target property for each of the second amount of polyethylene based blends;
- Setting a threshold for the one property related to the target property for polyethylene based blends to be selected for blending;
- Selecting the blends of the second amount of polyethylene based blends, at least one of which meet the set threshold of the predicted property related to the target property;
- Blending said selected blends.

Further, the present invention relates to polyethylene blends blended by the method as described above or below having a time to failure of at least 5 hours in full notch creep test (FNCT), determined according to ISO 16770 or a time to failure of at least 10 hours in environmental stress crack resistance (ESCR) test of bottle according to ASTM D2561.

Finally, the present invention relates to articles comprising the polyethylene blends as described above or below.

**Definitions**

The term "polyethylene based blend" is defined as the presence of at least two different polyethylenes such as two polyethylenes differing as to their density and/or molecular weight.

For example, a bimodal polyethylene as obtained from two reactors operated under different conditions constitutes a polyethylene blend, in this case an in-situ blend of two reactor products. Such in-situ blends or mechanical blends of two or more reactor products are called virgin blends in this invention.

It is self-explaining that polyethylene based blends obtained from consumer trash (i.e. post consumer waste) will include a broad variety of polyethylenes. In addition to that contamination by other plastics, mainly polypropylene, polystyrene, polyamide, polyesters, wood, paper, limonene, aldehydes, ketones, fatty acids, metals, and/or long term decomposition products of stabilizers can also be found. It goes without saying that such contaminants are not desirable.

Polyethylene based blends from post industrial waste usually includes less contaminants from non-polymeric material compared to post-consumer waste. Post industrial waste is usually collected from scrap, offcuts, off-spec. material, overproduction, rejects and other returns. The polyethylene based blends used in the method of the present invention can be virgin blends or recyclate blends.

The C2 fraction, which reflects the polyethylene content, can be measured by NMR of the d2-tetrachloroethylene soluble fraction. The percentage refer to the d2-tetrachloroethylene soluble part as used for the NMR experiment. The term "C2 fraction" equals the polymer fraction obtainable from ethylene monomer units, i.e., repetitive $-[C_2H_4]-$ units derived from ethylene which are present in the linear chains backbone and the short chain branches, whereby repetitive means at least two units.

The C2 fraction can be calculated as

$$wt_{C2fraction} = fC_{C2total} * 100 / (fC_{C2total} + fC_{PP})$$

whereby

$$fC_{C2total} = (Iddg - ItwoB4) + (IstarB1*6) + (IstarB2*7) + (ItwoB4*9) + (IthreeB5*10) +$$

$$((IstarB4plus - ItwoB4 - IthreeB5)*7) + (I3s*3)$$

and

$$fC_{PP} = Is\alpha\alpha * 3$$

The upper limit of the "C2 fraction" is 100 wt%. Details are given in the experimental part.

**Brief description of the figures**

Figure 1 shows the measured FNCT time to failure as a function of the predicted FNCT time to failure, which is calculated from $eta_{0.05}$ and $SHI_{2.7/210}$ via correlation (1).

Figure 2 shows the measured FNCT time to failure as a function of the predicted FNCT time to failure, which is calculated from $eta_{0.05}$ and PI via correlation (2).

Figure 3 shows the measured FNCT time to failure as a function of the predicted FNCT time to failure, which is calculated from $MFR_2$ and $FRR_{21/2}$ via correlation (3).

Figure 4 shows the measured time to failure of Bottle ESCR time to failure as a function of time to failure of FNCT.

**Detailed description**

In a first aspect the present invention relates to a method of blending polyethylene based blends comprising the steps of

- Providing a first amount of polyethylene based blends;
- Measuring properties of said first amount of polyethylene based blends, wherein at least one first property is related to the broadness of the molecular weight distribution curve of each of the first amount of polyethylene based blends, at least one second property is related to the molecular weight of each of the first amount of polyethylene based blends and one property related to the target property of a certain application for which the blended polyethylene based blends are intended to be used;
- Establishing a correlation between the at least one first property, the at least one second property and the one property related to the target property;
- Providing a second amount of polyethylene based blends;
- Measuring the same at least one first property and at least one second property, that were measured of the first amount of polyethylene based blends, of each of the second amount of polyethylene based blends;
- Using the correlation between the first property, the second property and the property related to the target property established for the first amount of polyethylene based blends for predicting the one property related to the target property for each of the second amount of polyethylene based blends;
- Setting a threshold for the one property related to the target property for polyethylene based blends to be selected for blending;
- Selecting the blends of the second amount of polyethylene based blends, at least one of which meet the set threshold of the predicted property related to the target property;
- Blending said selected blends.

The first amount of polyethylene based blends preferably includes 2 to 20, more preferably 3 to 15, more preferably 4 to 10 different polyethylene based blends.

"Different" in this case means that the blends originate from different sources.

The blends are preferably pre-selected for a specific application, such as blow moulding into containers or bottles, injection moulding into caps or extrusion into pipes. As a consequence the blends not necessarily differ to a great extent in their properties.

The first amount of polyethylene based blends can contain virgin polyethylene based blends and/or recycled polyethylene based blends.

In one embodiment the first amount of polyethylene based blends consists of virgin polyethylene based blends.

In a second embodiment the first amount of polyethylene based blends consists of recycled polyethylene based blends.

In a third embodiment the first amount of polyethylene based blends consists of virgin and recycled polyethylene based blends.

Recycled polyethylene based blends preferably are obtained from sorting processes for sorting post-consumer waste and/or post-industrial waste.

Sorting processes suitable for providing the recycled polyethylene based blends for the first amount of polyethylene based blends are known in the art. Exemplary sorting processes are e.g. described in Dr. Frank Welle, Develop a food grade HDPE recycling process, The Waste & Resources Action Programme, 4 June 2005 (received from https://www.researchgate.net/profile/Frank-Welle-2/publication/284158562_Develop_a_food_grade_HDPE_recycling_process/links/564ca7df0 8ae7ac727e20707/Develop-a-food-grade-HDPE-recycling-process.pdf?origin=publication_detail) or PCT/EP2022/064702.

The blends of the first amount of polyethylene based blends preferably consist of natural blends or white blends or a mix of natural and white blends. Natural blends are polyethylene based blends without filler or pigment, whereas white blends are polyethylene based blends with white filler or pigment. The white filler or pigment usually originates from titanium dioxide.

It is preferred that the first amount of polyethylene based blends does not include coloured or black polyethylene based blends.

Each blend of the first amount of polyethylene based blends irrespective of being a virgin blend or a recycled blend has one or more or all of the following properties:

Each blend preferably has a density of from 950 to 975 kg/m$^3$, more preferably from 952 to 972 kg/m$^3$, still more preferably from 955 to 970 kg/m$^3$, determined according to ISO 1183. The blends thus preferably all qualify as high density polyethylene (HDPE) blends.

Further, each blend preferably has a low amount of contaminants, preferably selected from chalk, talc, titanium dioxide, ash, polyamide, polystyrene and polypropylene, not more than 4.0 wt%, more preferably from 0 to 3.0 wt%, still more preferably from 0 to 2.5 wt%, based on the total weight of each blend, determined by FTIR.

Still further, each blend preferably has an ash content of not more than 3.0 wt%, preferably from 0 to 2.5 wt%, still more preferably from 0 to 2.0 wt%, based on the total weight of each blend, determined by TGA.

White blends usually have a higher ash content compared to natural blends.

White blends or mixed white and natural blends preferably have an ash content of not more than 3.0 wt%, preferably from 0.2 to 2.5 wt%, still more preferably from 0.3 to 2.0 wt%, based on the total weight of each blend, determined by TGA.

Natural blends preferably have an ash content of not more than 0.5 wt%, preferably from 0 to 0.3 wt%, still more preferably from 0 to 0.2 wt%, based on the total weight of each blend, determined by TGA.

Furthermore, each blend preferably has a titanium content of not more than 10,000 ppm, more preferably from 0 to 9,000 ppm, still more preferably from 0 to 8,500 ppm, based on the total weight of each blend, determined by XRF

White blends usually have a higher titanium content compared to natural blends.

White blends or mixed white and natural blends preferably have a titanium content of not more than 10,000 ppm, more preferably from 1,000 to 9,000 ppm, still more preferably from 2,500 to 8,500 ppm, based on the total weight of each blend, determined by XRF

Natural blends preferably have a titanium content of not more than 1,000 ppm, more preferably from 0 to 850 ppm, still more preferably from 0 to 750 ppm, based on the total weight of each blend, determined by XRF

Additionally, each blend preferably has an amount of polyethylene based polymer fraction of at least 97.0 wt%, more preferably from 97.0 to 100 wt%, still more preferably from 98.0 to 100 wt%, based on the total weight of each blend, determined by NMR.

Further, each blend preferably has an amount of ethylene monomer units in the polyethylene based polymer fraction of from 98.0 to 100 wt%, more preferably from 98.5 to 100 wt%, still more preferably from 99.0 to 100 wt%, based on the total weight of the polyethylene based polymer fraction, determined by NMR.

Still further, each blend preferably has an amount of alpha-olefin comonomer units in the polyethylene based polymer fraction of from 0 to 2.0 wt%, more preferably from 0 to 1.5 wt%, still more preferably from.0 to 1.0 wt%, based on the total weight of the polyethylene based polymer fraction, determined by NMR.

In a second step, properties of said first amount of polyethylene based blends are measured for each of the blends.

For example, each blend of the first amount of polyethylene based blends used for preparing a polyethylene based blend for bottles or containers preferably has one or more or all of the following mechanical properties:

Each blend preferably has a melt flow rate $MFR_2$ of from 0.01 to 2.5 g/10 min, more preferably from 0.05 to 2.0 g/10 min, still more preferably from 0.1 to 1.5 g/10 min, determined according to ISO 1133 at 190°C and 2.16 kg.

Further, each blend preferably has a melt flow rate $MFR_5$ of from 0.1 to 5.0 g/10 min, more preferably from 0.5 to 4.0 g/10 min, still more preferably from 1.0 to 3.0 g/10 min, determined according to ISO 1133 at 190°C and 5 kg.

Still further, each blend preferably has a melt flow rate $MFR_{21}$ of from 20 to 55 g/10 min, more preferably from 22 to 50 g/10 min, still more preferably from 25 to 45 g/10 min, determined according to ISO 1133 at 190°C and 21.6 kg.

Additionally, each blend preferably has a flow rate ratio $FRR_{21/2}$, being the ratio of $MFR_{21}$ to $MFR_2$, of from 70 to 110, more preferably from 75 to 100, still more preferably from 80 to 95.

Furthermore, each blend preferably has a flow rate ratio $FRR_{21/5}$, being the ratio of $MFR_{21}$ to $MFR_5$, of from 10 to 30, more preferably from 12 to 27, more preferably from 15 to 25.

Further, each blend preferably has a polydispersity index PI of from 1.5 to 3.5 $s^{-1}$, more preferably from 2.2 to 3.2 $s^{-1}$, still more preferably from 2.0 to 3.0 $s^{-1}$, determined from dynamic shear measurements.

Still further, each blend preferably has a shear thinning index $SHI_{2.7/210}$ of from 25 to 50, more preferably from 27 to 47, still more preferably 30 to 45, determined from dynamic shear measurements.

Additionally, each blend preferably has a complex viscosity at a frequency of 0.05 rad/s $etao.os$ of from 25,000 to 55,000 Pa·s, more preferably from 27,500 to 52,500 Pa·s, still more preferably from 30,000 to 50,000 Pa·s, determined from dynamic shear measurements.

Furthermore, each blend preferably has a complex viscosity at a frequency of 300 rad/s $eta_{300}$ of from 500 to 1000 Pa·s, more preferably from 600 to 900 Pa·s, still more preferably from 650 to 850 Pa·s, determined from dynamic shear measurements.

Further, each blend preferably has a flexural modulus of from 750 to 1500 MPa, more preferably from 800 to 1250 MPa, still more preferably from 850 to 1050 MPa, determined according to ISO 178.

Still further, each blend preferably has an FNCT time to failure of from 2.0 to 2000 hours, more preferably from 2.2 to 1500 hours, still more preferably from 2.5 to 1000 hours, determined according ISO 16770.

Thereby, recycled polyethylene based blends typically have a lower FNCT time to failure compared to virgin polyethylene based blends.

When using only recycled polyethylene based blends the FNCT time to failure is usually from 2.0 to 500 hours, more preferably from 2.2 to 350 hours, still more preferably from 2.5 to 250 hours, determined according ISO 16770.

When using only virgin polyethylene based blends the FNCT time to failure is usually from 250 to 2000 hours, more preferably from 350 to 1500 hours, still more preferably from 500 to 1000 hours, determined according ISO 16770.

Depending on the application of the polyethylene blend blended by the method according to the invention the blends of the first amount of polyethylene based blends are preferably pre-selected in regard of their mechanical properties so that blends meeting one or more or preferably all of the above cited properties are met.

From the measured properties of the first amount of polyethylene based blends at least one first property is related to the broadness of the molecular weight distribution curve of each of the first amount of polyethylene based blends, at least one second property is related to the molecular weight of each of the first amount of polyethylene based blends and one property related to the target property of a certain application for which the blended polyethylene based blends are intended to be used are selected.

The at least one first property related to the broadness of the molecular weight distribution curve can be

1) parameter(s) determined from dynamic shear measurements, such as shear thinning index $SHI_{1/100}$, $SHI_{2.7/210}$, $SHI_{5/300}$, polydispersity index PI,
2) parameter(s) determined according to ISO 1133, such as flow rate ratio $FRR_{21/2}$, flow rate ratio $FRR_{21/5}$,
3) parameter(s) determined from GPC measurement, such as Mw/Mn, Mz/Mw,
4) and combinations thereof,

or other suitable properties.

The at least one second property related to the related to the molecular weight of each of the first amount of polyethylene based blends can be

1) parameter(s) determined from dynamic shear measurements, such as $eta_{0.05}$, $eta_{300}$,
2) parameter(s) determined according to ISO 1133, such as $MFR_2$, $MFR_5$, $MFR_{21}$,
3) parameter(s) determined from GPC measurement, such as Mw, Mz,
4) and combinations thereof,

or other suitable properties.

The property related to the target property of a certain application are usually properties, which are related to the long term stability of the end application. Examples are e.g. FNCT time to failure, ESCR, pipe pressure tests such as according to ISO 1167 or ISO 13479 or other properties related to long term stability.

These properties usually are conducted over a long time and therefore are desirable for prediction.

After measurement of the properties a correlation between the at least one first property, the at least one second property and the one property related to the target property is established.

Such correlations can be established in a manner known in the art, e.g. by establishing trendlines in suitable mathematical computer programs for the three desired properties.

Preferably the correlation is established by multiple linear regression.

The correlation for the properties of $eta_{0.05}$, $SHI_{2.7/210}$ and FNCT time to failure is preferably

$$\text{Pred. FNCT time to failure (h)} = a + b \cdot eta_{0.05} \text{ (Pa·s) - } c \cdot SHI_{2.7/210} \tag{1}$$

with

Pred. FNCT time to failure being the FNCT time to failure predicted by correlation (1);
a being in the range of from 30.000 to 48.000, preferably from 33.000 to 45.000;
b being in the range of from 5.000E-4 to 7.500E-4, preferably from 5.500E-4 to 7.000E-4; and
c being in the range of from 1.300 to 1.600, preferably from 1.400 to 1.480.

For example for the properties of $eta_{0.os}$, $SHI_{2.7/210}$ and FNCT time to failure the following correlation (1) has been established from the polyethylene based blends of examples PE1-PE7 listed in the example section below:

$$\text{Pred. FNCT time to failure (h)} = 38.962 + 6.806E\text{-}4 \cdot eta_{0.05} \text{ (Pa·s) - } 1.438 \cdot SHI_{2.7/210} \tag{1a}$$

The correlation for the properties of $eta_{0.05}$, PI and FNCT time to failure is preferably

$$\text{Pred. FNCT time to failure (h)} = d + e \cdot eta_{0.05} \text{ (Pa·s) - } f \cdot PI \text{ (1/Pa)} \tag{2}$$

with

Pred. FNCT time to failure being the FNCT time to failure predicted by correlation (2);
d being in the range of from 65.000 to 90.000, preferably from 67.500 to 88.000;
e being in the range of from 0.00050 to 0.00200, preferably from 0.00100 to 0.00150; and
f being in the range of from 40.000 to 55.000, preferably from 45.000 to 52.000.

For example for the properties of $eta_{0.05}$, PI and FNCT time to failure the following correlation (2) has been established from the polyethylene based blends of examples PE1-PE7 listed in the example section below:

$$\text{Pred. FNCT time to failure (h)} = 69.915 + 0.0013 \cdot eta_{0.05} \text{ (Pa·s) - } 46.949 \cdot PI \text{ (1/Pa)} \tag{2a}$$

The correlation for the properties of $MFR_2$, $FRR_{21/2}$ and FNCT time to failure is preferably time to failure

$$\text{Pred. FNCT time to failure (h)} = g + h \cdot MFR_2 \text{ (g/10 min) - } i \cdot FRR_{21/2} \tag{3}$$

with

Pred. FNCT time to failure being the FNCT time to failure predicted by correlation (3);
g being in the range of from 265.000 to 400.000, preferably from 330.000 to 390.000;
h being in the range of from 140.000 to 165.000, preferably from 150.000 to 162.500; and
i being in the range of from 2.500 to 4.000, preferably from 2.750 to 3.750.

For example for the properties of $MFR_2$, $FRR_{21/2}$ and FNCT time to failure the following correlation (3) has been established from the polyethylene based blends of examples PE1-PE7 listed in the example section below:

$$\text{Pred. FNCT time to failure (h)} = 335.812 - 153.036 \cdot MFR_2 \text{ (g/10 min) - } 3.018 \cdot FRR_{21/2} \tag{3a}$$

In a further step, a second amount of polyethylene based blends is provided.

The second amount of polyethylene based blends preferably includes 2 to 20, more preferably 3 to 15, more preferably 4 to 10 different polyethylene based blends.

"Different" in this case means that the blends originate from different sources.

The second amount of polyethylene based blends can contain virgin polyethylene based blends and/or recycled polyethylene based blends.

In one embodiment the second amount of polyethylene based blends consists of virgin polyethylene based blends.

In a second embodiment the second amount of polyethylene based blends consists of recycled polyethylene based blends.

In a third embodiment the second amount of polyethylene based blends consists of virgin and recycled polyethylene based blends.

Recycled polyethylene based blends preferably are obtained from sorting processes for sorting post-consumer waste and/or post-industrial waste.

Sorting processes suitable for providing the recycled polyethylene based blends for the first amount of polyethylene based blends are known in the art. Examplary sorting processes are e.g. described in Dr. Frank Welle, Develop a food grade HDPE recycling process, The Waste & Resources Action Programme, 4 June 2005 (received from https://www.researchgate.net/profile/Frank-Welle-2/publication/284158562_Develop_a_food_grade_HDPE_recycling_process/links/564ca7df0 8ae7ac727e20707/Develop-a-food-grade-HDPE-recycling-process.pdf?origin=publication_detail) or PCT/EP2022/064702.

The blends of the second amount of polyethylene based blends preferably consist of natural blends or white blends or a mix of natural and white blends. Natural blends are polyethylene based blends without filler or pigment, whereas white blends are polyethylene based blends with white filler or pigment. White filler or pigment usually originates from titanium dioxide. It is preferred that the second amount of polyethylene based blends does not include coloured or black polyethylene based blends.

Each blend of the second amount of polyethylene based blends irrespective of being a virgin blend or a recycled blend has one or more or all of the following properties:

Each blend preferably has a density of from 950 to 975 kg/m$^3$, more preferably from 952 to 972 kg/m$^3$, still more preferably from 955 to 970 kg/m$^3$, determined according to ISO 1183. The blends thus preferably all qualify as high density polyethylene (HDPE) blends.

Further, each blend preferably has an amount of contaminants, preferably selected from chalk, talc, titanium dioxide, ash, polyamide, polystyrene and polypropylene, not more than 4.0 wt%, more preferably from 0 to 3.0 wt%, still more preferably from 0 to 2.5 wt%, based on the total weight of each blend, determined by FTIR.

Still further, each blend preferably has an ash content of not more than 3.0 wt%, preferably from 0 to 2.5 wt%, still more preferably from 0 to 2.0 wt%, based on the total weight of each blend, determined by TGA.

White blends usually have a higher ash content compared to natural blends.

White blends or mixed white and natural blends preferably have an ash content of not more than 3.0 wt%, preferably from 0.2 to 2.5 wt%, still more preferably from 0.3 to 2.0 wt%, based on the total weight of each blend, determined by TGA.

Natural blends preferably have an ash content of not more than 0.5 wt%, preferably from 0 to 0.3 wt%, still more preferably from 0 to 0.2 wt%, based on the total weight of each blend, determined by TGA.

Furthermore, each blend preferably has a titanium content of not more than 10,000 ppm, more preferably from 0 to 9,000 ppm, still more preferably from 0 to 8,500 ppm, based on the total weight of each blend, determined by XRF

White blends usually have a higher titanium content compared to natural blends.

White blends or mixed white and natural blends preferably have a titanium content of not more than 10,000 ppm, more preferably from 1,000 to 9,000 ppm, still more preferably from 2,500 to 8,500 ppm, based on the total weight of each blend, determined by XRF

Natural blends preferably have a titanium content of not more than 1,000 ppm, more preferably from 0 to 850 ppm, still more preferably from 0 to 750 ppm, based on the total weight of each blend, determined by XRF

Additionally, each blend preferably has an amount of polyethylene based polymer fraction of at least 97.0 wt%, more preferably from 97.0 to 100 wt%, still more preferably from 98.0 to 100 wt%, based on the total weight of each blend, determined by NMR.

Further, each blend preferably has an amount of ethylene monomer units in the polyethylene based polymer fraction of from 98.0 to 100 wt%, more preferably from 98.5 to 100 wt%, still more preferably from 99.0 to 100 wt%, based on the total weight of the polyethylene based polymer fraction, determined by NMR.

Still further, each blend preferably has an amount of alpha-olefin comonomer units in the polyethylene based polymer fraction of from 0 to 2.0 wt%, more preferably from 0 to 1.5 wt%, still more preferably from 0 to 1.0 wt%, based on the total weight of the polyethylene based polymer fraction, determined by NMR.

In a second step, properties of said second amount of polyethylene based blends related to the first property and the second property used for the correlation between the at least one first property, the at least one second property and the one property related to the target property established from the first amount of polyethylene based blends are measured for each of the blend.

Each blend preferably has a melt flow rate $MFR_2$ of from 0.01 to 2.5 g/10 min, more preferably from 0.05 to 2.0 g/10 min, still more preferably from 0.1 to 1.5 g/10 min, determined according to ISO 1133 at 190°C and 2.16 kg.

Further, each blend preferably has a melt flow rate $MFR_5$ of from 0.1 to 5.0 g/10 min, more preferably from 0.5 to 4.0 g/10 min, still more preferably from 1.0 to 3.0 g/10 min, determined according to ISO 1133 at 190°C and 5 kg.

Still further, each blend preferably has a melt flow rate $MFR_{21}$ of from 20 to 55 g/10 min, more preferably from 22 to 50 g/10 min, still more preferably from 25 to 45 g/10 min, determined according to ISO 1133 at 190°C and 21.6 kg.

Additionally, each blend preferably has a flow rate ratio $FRR_{21/2}$, being the ratio of $MFR_{21}$ to $MFR_2$, of from 70 to 110, more preferably from 75 to 100, still more preferably from 80 to 95.

Furthermore, each blend preferably has a flow rate ratio $FRR_{21/5}$, being the ratio of $MFR_{21}$ to $MFR_5$, of from 10 to 30, more preferably from 12 to 27, more preferably from 15 to 25.

Further, each blend preferably has a polydispersity index PI of from 1.5 to 3.5 $s^{-1}$, more preferably from 2.2 to 3.2 $s^{-1}$, still more preferably from 2.0 to 3.0 $s^{-1}$, determined from dynamic shear measurements.

Still further, each blend preferably has a shear thinning index $SHI_{2.7/210}$ of from 25 to 50, more preferably from 27 to 47, still more preferably 30 to 45, determined from dynamic shear measurements.

Additionally, each blend preferably has a complex viscosity at a frequency of 0.05 rad/s etao.os of from 25,000 to 55,000 Pa·s, more preferably from 27,500 to 52,500 Pa·s, still more preferably from 30,000 to 50,000 Pa·s, determined from dynamic shear measurements.

Furthermore, each blend preferably has a complex viscosity at a frequency of 300 rad/s $eta_{300}$ of from 500 to 1000 Pa·s, more preferably from 600 to 900 Pa·s, still more preferably from 650 to 850 Pa·s, determined from dynamic shear measurements.

Further, each blend preferably has a flexural modulus of from 750 to 1500 MPa, more preferably from 800 to 1250 MPa, still more preferably from 850 to 1050 MPa, determined according to ISO 178.

The correlation is then used for predicting the one property related to the target property for each of the second amount of polyethylene based blends.

For example for correlation (1) $eta_{0.05}$ and $SHI_{2.7/210}$ are measured for each blend of the second amount of polyethylene based blends and FNCT time to failure is predicted by calculation using correlation (1).

For example for correlation (2) $eta_{0.05}$ and PI are measured for each blend of the second amount of polyethylene based blends and FNCT time to failure is predicted by calculation using correlation (2).

For example for correlation (3) $MFR_2$ and $FRR_{21/2}$ are measured for each blend of the second amount of polyethylene based blends and FNCT time to failure is predicted by calculation using correlation (3).

For selecting individual blends from the second amount of polyethylene based blends a threshold for the one property related to the target property is set. By setting such a threshold it is ensured that at least one blend, preferably more than one blend, most preferably only blends from the second amount of polyethylene based blends are used for the final blending which show good predicted behaviour in said target property.

In a further step at least one blend, preferably more than one blend, most preferably only the individual blends from the second amount of polyethylene based blends which have a predicted target property as calculated from the correlation above the set threshold are selected for the final blending step.

The selected blends from the second amount of polyethylene based blends are then blended to obtain the final polyethylene blend which is then used for producing the intended article.

Preferably the target property is verified in said final blend by measurement on the final polyethylene blend.

When used for bottles or containers the final polyethylene blend preferably has a time to failure of at least 5 hours, more preferably of at least 10 hours in full notch creep test (FNCT), determined according to ISO 16770.

The upper limit usually does not exceed 120 hours.

When used for bottles or containers the final polyethylene blend preferably has a time to failure of at least 10 hours, preferably of at least 15 hours in environmental stress crack resistance (ESCR) test of bottle according to ASTM D2561.

The upper limit usually does not exceed 180 hours.

The present invention preferably is also related to a polyethylene blend produced by the method according to the present invention.

Thereby, all aspects of the method as described above and below and the polyethylene blend as described above or below preferably apply to the polyethylene blend.

Still further, the present invention also relates to an article comprising the polyethylene blend produced by the method according to the present invention.

Thereby, all aspects of the method as described above and below and the polyethylene based blend as described above or below preferably apply to the article.

The article preferably is a bottle or container.

The bottle or container preferably is produced by means of blow moulding. Blow moulding processes are known in the art.

Finally, the present invention relates to the method as described above or below for producing polyethylene blends having a time to failure of at least 5 hours in full notch creep test (FNCT), determined according to ISO 16770 or a time to failure of at least 10 hours in environmental stress crack resistance (ESCR) test of bottle according to ASTM D2561.

Thereby, all aspects of the method as described above and below and the polyethylene blend as described above or below preferably apply to the use.

## Experimental

The following Examples are included to demonstrate certain aspects and embodiments of the invention as described in the claims. It should be appreciated by those of skill in the art, however, that the following description is illustrative only and should not be taken in any way as a restriction of the invention.

Test Methods

a) MFR

Melt flow rates were measured at a temperature of 190 °C and different loads of 2.16 kg ($MFR_2$), of 5.00 kg ($MFR_5$) and of 21.6 kg ($MFR_{21}$). The melt flow rate is that quantity of polymer in grams which the test apparatus standardized to ISO 1133 extrudes within 10 minutes at a temperature of 190 °C under the above stated loads.

**b) Density**

According to ISO 1183-1. Sample preparation is done by compression moulding in accordance with ISO 17855-2.

**c) C2 fraction by NMR spectroscopy and general microstructure including "continuous C3" as well as short chain branches**

Quantitative $^{13}$C{$^1$H} NMR spectra were recorded in the solution-state using a Bruker AVNEO 400MHz NMR spectrometer operating at 400.15 and 100.62 MHz for $^1$H and $^{13}$C respectively. All spectra were recorded using a $^{13}$C optimised 10 mm extended temperature probehead at 125°C using nitrogen gas for all pneumatics. Approximately 200 mg of material was dissolved in approximately 3 ml of *1,2*-tetrachloroethane-$d_2$ (TCE-$d_2$) along with approximately 3 mg BHT (2,6-di-tert-butyl-4-methylphenol CAS 128-37-0) and chromium-(III)-acetylacetonate (Cr(acac)$_3$) resulting in a 60 mM solution of relaxation agent in solvent {singh09}. To ensure a homogenous solution, after initial sample preparation in a heat block, the NMR tube was further heated in a rotatory oven for at least 1 hour. Upon insertion into the magnet the tube was spun at 10 Hz. Standard single-pulse excitation was employed without NOE, using an optimised tip angle, 1 s recycle delay and a bi-level WALTZ 16 decoupling scheme {zhou07,busico07}. A total of 6144 (6k) transients were acquired per spectra. Quantitative $^{13}$C{$^1$H} NMR spectra were processed, integrated and relevant quantitative properties determined from the integrals using proprietary computer programs. All chemical shifts were indirectly referenced to the central methylene group of the ethylene block (EEE) at 30.00 ppm using the chemical shift of the solvent. Characteristic signals corresponding to polyethylene with different short chain branches (B1, B2, B4, B5, B6plus) and polypropylene were observed {randall89, brandolini00}.

Characteristic signals corresponding to the presence of polyethylene containing isolated B1 branches (starB1 33.3 ppm), isolated B2 branches (starB2 39.8 ppm), isolated B4 branches (twoB4 23.4 ppm), isolated B5 branches (threeB5 32.8 ppm), all branches longer than 4 carbons (starB4plus 38.3 ppm) and the third carbon from a saturated aliphatic chain end (3s 32.2 ppm) were observed. If one or the other structural element is not observable it is excluded from the equations. The intensity of the combined ethylene backbone methine carbons (ddg) containing the polyethylene backbone carbons (dd 30.0 ppm), γ-carbons (g 29.6 ppm) the 4s and the threeB4 carbon (to be compensated for later on) is taken between 30.9 ppm and 29.3 ppm excluding the Tββ from polypropylene. The amount of C2 related carbons was quantified using all mentioned signals according to the following equation:

$$fC_{C2total} = (Iddg - ItwoB4) + (IstarB1*6) + (IstarB2*7) + (ItwoB4*9) + (IthreeB5*10) +$$

$$((IstarB4plus-ItwoB4-IthreeB5)*7) + (I3s*3)$$

When characteristic signals corresponding to the presence of polypropylene (PP, continuous C3) were observed at 46.7 ppm, 29.0 ppm and 22.0 ppm the amount of PP related carbons was quantified using the integral of $S\alpha\alpha$ at 46.6 ppm:

$$fC_{PP} = Is\alpha\alpha * 3$$

The weight percent of the C2 fraction and the polypropylene can be quantified according following equations:

$$wt_{C2fraction} = fC_{C2total} * 100 / (fC_{C2total} + fC_{PP})$$

$$wt_{PP} = fC_{PP} * 100 / (fC_{C2total} + fC_{PP})$$

Characteristic signals corresponding to various short chain branches were observed and their weight percentages quantified as the related branch would be an alpha-olefin, starting by quantifying the weight fraction of each:

$$fwtC2 = fC_{C2total} - (IstarB1*3) - (IstarB2*4) - (ItwoB4*6) - (IthreeB5*7)$$

$$fwtC3 \ (isolated \ C3) = IstarB1*3$$

$$fwtC4 = IstarB2*4$$

$$fwtC6 = ItwoB4*6$$

$$fwtC7 = IthreeB5*7$$

Normalisation of all weight fractions leads to the amount of weight percent for all related branches:

$$fsum_{wt\%total} = fwtC2 + fwtC3 + fwtC4 + fwtC6 + fwtC7 + fC_{PP}$$

$$wtC2total = fwtC2 * 100 / fsum_{wt\%total}$$

$$wtC3total = fwtC3 * 100 / fsum_{wt\%total}$$

$$wtC4total = fwtC4 * 100 / fsum_{wt\%total}$$

$$wtC6total = fwtC6 * 100 / fsum_{wt\%total}$$

$$wtC7total = fwtC7 * 100 / fsum_{wt\%total}$$

References:

zhou07 Zhou, Z., Kuemmerle, R., Qiu, X., Redwine, D., Cong, R., Taha, A., Baugh, D. Winniford, B., J. Mag. Reson.

187 (2007) 225

busico07 Busico, V., Carbonniere, P., Cipullo, R., Pellecchia, R., Severn, J., Talarico, G., Macromol. Rapid Commun. 2007, 28, 1128

singh09 Singh, G., Kothari, A., Gupta, V., Polymer Testing 28 5 (2009), 475

randall89 J. Randall, Macromol. Sci., Rev. Macromol. Chem. Phys. 1989, C29, 201.

brandolini00 A. J. Brandolini, D. D. Hills, NMR Spectra of Polymers and Polymer Additives, Marcel Dekker Inc., 2000

**d) OCS gels and contaminations**

The gel count was measured with a gel counting apparatus consisting of a measuring extruder, ME 25 / 5200 VI, 25*25D, with five temperature conditioning zones adjusted to a typical temperature profile of 190/200/210/210/200°C), an adapter and a slit die (with an opening of 0.5 * 150 mm). Attached to this were a chill roll unit (with a diameter of 13 cm with a temperature set of 50°C), a line camera (CCD 4096 pixel for dynamic digital processing of grey tone images) and a winding unit.

For the gel count content measurements the materials were extruded at a screw speed of 30 rounds per minute, a drawing speed of 3-3.5 m/min and a chill roll temperature of 50°C to make thin cast films with a thickness of 70 $\mu$m and a width of approximately 110 mm.

The resolution of the camera is 25 $\mu$m $\times$ 25 $\mu$m on the film.

The camera works in transmission mode with a constant grey value (auto. set. margin level = 170) by using one of two different sensitivity levels to distinguish between Gels and Contaminations.

The system is able to decide between 256 grey values from black = 0 to white = 256.

> 1 level dark: 25% (Gels)
> 2 level dark: 10% (Contaminations)

For each material the average number of gel dots on a film surface area of 10 m² was inspected by the line camera. The number of contaminations are detected in the same way. The line camera was set to differentiate the gel/contamination size according to the following:
Gel/contamination size

> 100 $\mu$m to 299 $\mu$m
> 300 $\mu$m to 599 $\mu$m
> 600 $\mu$m to 999 $\mu$m
> above 1000 $\mu$m

**e) Evaluation of recycled nature**

**fa) Elements**

The atomic elements are determined by x ray fluorescence (XRF).

**fb) Amount of "iPP" and other components determination by Transmission Infra-Red spectroscopy**

Determination of the components and their amount in the recycled polymer composition is accomplished by FTIR-spectroscopy:
Sample preparation:
All calibration samples and samples to be analyzed were prepared in similar way, on molten pressed plates.

Around 2 to 3 g of compounds to be analyzed were molten at 190°C. Subsequently, for 20 seconds 60 to 80 bar pressure was applied in a hydraulic heating press. Next, the samples were cooled down to room temperature in 40 second in a cold press under the same pressure, in order to control the morphology of the compound. The thickness of the plates were controlled by metallic calibrated frame plates 2.5 cm by 2.5 cm, 100 to 200 $\mu$m thick (depending MFR from the sample); two plates were produced in parallel at the same moment and in the same conditions. The thickness of each plate was measured before any FTIR measurements; all plates were between 100 to 200 $\mu$m thick.

To control the plate surface and to avoid any interference during the measurement, all plates were pressed between two double-sided silicone release papers.

In case of powder samples or heterogeneous compounds, the pressing process would be repeated three times to increase homogeneity by pressed and cutting the sample in the same conditions as described before.

Spectrometer:

Standard transmission FTIR spectroscope such as Bruker Vertex 70 FTIR spectrometer was used with the following set-up:

- a spectral range of 4000-400 cm$^{-1}$,
- an aperture of 6 mm,
- a spectral resolution of 2 cm$^{-1}$,
- with 16 background scans, 16 spectrum scans,
- an interferogram zero filling factor of 32
- Norton Beer strong apodisation.

Spectrum are recorded and analysed in Bruker Opus software.

Calibration samples:
As FTIR is a secondary method, several calibration standards were compounded to cover the targeted analysis range, typically from:

- 0.2 wt% to 2.5 wt% for PA
- 0.1 wt% to 5 wt% for PS
- 0.2 wt% to 2.5 wt% for PET
- 0.1 wt% to 4 wt% for PVC

The following commercial materials were used for the compounds: Borealis HC600TF as iPP, Borealis FB3450 as HDPE and for the targeted polymers such RAMAPET N1S
(Indorama Polymer) for PET, Ultramid® B36LN (BASF) for Polyamide 6, Styrolution PS 486N (Ineos) for High Impact Polystyrene (HIPS), and for PVC Inovyn PVC 263B (under powder form).

All compounds are made at small scale in a Haake kneader at a temperature below 265°C and less than 10 minutes to avoid degradation.

Additional antioxidant such as Irgafos 168 (3000 ppm) is added to minimize the degradation.

Calibration:
The FTIR calibration principal is the same for all the components: the intensity of a specific FTIR band divided by the plate thickness is correlated to the amount of component determined by $^1$H or $^{13}$C solution state NMR on the same plate.

Each specific FTIR absorption band is chosen due to its intensity increase with the amount of the component concentration and due to its isolation from the rest of the peaks, whatever the composition of the calibration standard and real samples.

This methodology is described in the publication from Signoret and al. "Alterations of plastic spectra in MIR and the potential impacts on identification towards recycling", Resources, conservation and Recycling journal, 2020, volume 161, article 104980.

The wavelength for each calibration band is:

- 3300 cm$^{-1}$ for PA,
- 1601 cm$^{-1}$ for PS,
- 1410 cm$^{-1}$ for PET,
- 615 cm$^{-1}$ for PVC,
- 1167 cm$^{-1}$ for iPP.

For each polymer component i, a linear calibration (based on linearity of Beer-Lambert law) is constructed. A typical linear correlation used for such calibrations is given below:

$$x_i = A_i \cdot \frac{E_i}{d} + B_i$$

where

$x_i$ is the fraction amount of the polymer component i (in wt%)
Ei is the absorbance intensity of the specific band related to the polymer component
i (in a.u. absorbance unit). These specific bands are, 3300 cm$^{-1}$ for PA, 1601 cm$^{-1}$
for PS, 1410 cm$^{-1}$ for PET, 615 cm$^{-1}$ for PVC, 1167 cm$^{-1}$ for iPP
d is the thickness of the sample plate

$A_i$ and $B_i$ are two coefficients of correlation determined for each calibration curve

The following bands are used to estimate the EVA, Chalk and Talc contents:

EVA: band centred at 607 cm$^{-1}$
Chalk : band centred at 1798 cm$^{-1}$
Talc : band centred at 3676 cm$^{-1}$

For each calibration standard, wherever available, the amount of each component is determined by either $^1$H or $^{13}$C solution state NMR, as primary method (except for PA). The NMR measurements are performed on the exact same FTIR plates used for the construction of the FTIR calibration curves.

**fc) ash**

Thermogravimetric Analysis (TGA) experiments were performed with a Perkin Elmer TGA 8000. Approximately 10-20 mg of materials were placed in a platinum pan. The temperature was equilibrated at 50°C for 10 minutes, and afterwards raised to 950°C under nitrogen at 20°C/min. The ash content was evaluated as the weight % at 900 °C.

**fd) water content**

The water content was determined as described in ISO15512:2019 Method A - Extraction with anhydrous methanol. There the test portion is extracted with anhydrous methanol and the extracted water is determined by a coulometric Karl Fischer Titrator.

**f) Rheological measurements**

Dynamic Shear Measurements (frequency sweep measurements)

The characterisation of melt of polymer composition or polymer as given above or below in the context by dynamic shear measurements complies with ISO standards 6721-1 and 6721-10. The measurements were performed on an Anton Paar MCR501 stress controlled rotational rheometer, equipped with a 25 mm parallel plate geometry. Measurements were undertaken on compression moulded plates, using nitrogen atmosphere and setting a strain within the linear viscoelastic regime. The oscillatory shear tests were done at 190 °C applying a frequency range between 0.01 and 600 rad/s and setting a gap of 1.3 mm.

In a dynamic shear experiment the probe is subjected to a homogeneous deformation at a sinusoidal varying shear strain or shear stress (strain and stress controlled mode, respectively). On a controlled strain experiment, the probe is subjected to a sinusoidal strain that can be expressed by

$$\gamma(t) = \gamma_0 \sin(\omega t) \qquad (1)$$

If the applied strain is within the linear viscoelastic regime, the resulting sinusoidal stress response can be given by

$$\sigma(t) = \sigma_0 \sin(\omega t + \delta) \qquad (2)$$

where

$\sigma_0$ and $\gamma_0$ are the stress and strain amplitudes, respectively
$\omega$ is the angular frequency
$\delta$ is the phase shift (loss angle between applied strain and stress response)
t is the time

Dynamic test results are typically expressed by means of several different rheological functions, namely the shear storage modulus G', the shear loss modulus, G", the complex shear modulus, G*, the complex shear viscosity, $\eta^*$, the dynamic shear viscosity, $\eta'$, the out-of-phase component of the complex shear viscosity $\eta''$ and the loss tangent, tan $\delta$ which can be expressed as follows:

$$G' = \frac{\sigma_0}{\gamma_0} \cos\delta \; [\text{Pa}] \tag{3}$$

$$G'' = \frac{\sigma_0}{\gamma_0} \sin\delta \; [\text{Pa}] \tag{4}$$

$$G^* = G' + iG'' \; [\text{Pa}] \tag{5}$$

$$\eta^* = \eta' - i\eta'' \; [\text{Pa·s}] \tag{6}$$

$$\eta' = \frac{G''}{\omega} \; [\text{Pa·s}] \tag{7}$$

$$\eta'' = \frac{G'}{\omega} \; [\text{Pa·s}] \tag{8}$$

The determination of so-called Shear Thinning Index, which correlates with MWD and is independent of Mw, is done as described in equation 9.

$$\text{SHI}_{(x/y)} = \frac{\text{Eta* for } (G^* = x \text{ kPa})}{\text{Eta* for } (G^* = y \text{ kPa}} \tag{9}$$

For example, the $\text{SHI}_{(2.7/210)}$ is defined by the value of the complex viscosity, in Pa·s, determined for a value of $G^*$ equal to 2.7 kPa, divided by the value of the complex viscosity, in Pa·s, determined for a value of $G^*$ equal to 210 kPa.

The values of storage modulus (G'), loss modulus (G"), complex modulus (G*) and complex viscosity ($\eta^*$) were obtained as a function of frequency ($\omega$).

Thereby, e.g. $\eta^*_{300\text{rad/s}}$ (eta$^*_{300\text{rad/s}}$ or eta$_{300}$) is used as abbreviation for the complex viscosity at the frequency of 300 rad/s and $\eta^*_{0.05\text{rad/s}}$ (eta$^*_{0.05\text{rad/s}}$ or eta$_{0.05}$) is used as abbreviation for the complex viscosity at the frequency of 0.05 rad/s.

The polydispersity index, *PI,* is defined by equation 10.

$$PI = \frac{10^5}{G'(\omega_{COP})}, \qquad \omega_{COP} = \omega \text{ for } (G' = G'') \tag{10}$$

where $\omega_{COP}$ is the cross-over angular frequency, determined as the angular frequency for which the storage modulus, G', equals the loss modulus, G".

The values are determined by means of a single point interpolation procedure, as defined by Rheoplus software. In situations for which a given G* value is not experimentally reached, the value is determined by means of an extrapolation, using the same procedure as before. In both cases (interpolation or extrapolation), the option from Rheoplus *"Interpolate y-values to x-valuesfrom parameter"* and the *"logarithmic interpolation type"* were applied.

References:

[1] Rheological characterization of polyethylene fractions" Heino, E.L., Lehtinen, A., Tanner J., Seppälä, J., Neste Oy, Porvoo, Finland, Theor. Appl. Rheol., Proc. Int. Congr. Rheol, 11th (1992), 1, 360-362
[2] The influence of molecular structure on some rheological properties of polyethylene", Heino, E.L., Borealis Polymers Oy, Porvoo, Finland, Annual Transactions of the Nordic Rheology Society, 1995.).
[3] Definition of terms relating to the non-ultimate mechanical properties of polymers, Pure & Appl. Chem., Vol. 70, No. 3, pp. 701-754, 1998.

**g) XHU**

The xylene hot insoluble amount (XHU) was analyzed according to ISO 10147. Therefore, 1 g of the sample is weighed out exactly to 0.1 mg ($m_1$) and placed in a pouch made of stainless steel mesh of the following material ("stainless steal quality 1.4401"). The pouch with polymer sample was weighed out exactly to 0.1 mg ($m_2$), and placed in a round flask filled with 700 mL xylene (ortho- or para-xylene, with a purity chromatography >98%). The xylene was kept under reflux conditions for 5h. The pouch containing the insoluble matter was taken out of the flask, washed from the polymer solution residues with a fresh portion of 700 mL xylene under reflux conditions for another 30 minutes. Afterwards the pouch was taken out of the flask, dried under vacuum at 90 °C to constant mass. After cooled to room temperature in a desiccator, the pouch was weighed out exactly to the nearest 0.1 mg ($m_3$).

$$\%XHU = \frac{m_3 - (m_2 - m_1)}{m_1} * 100$$

XHU = Xylene hot insoluble amount
$m_1$ = sample weight in g
$m_2$ = weight of pouch with the sample in g
$m_3$ = weight of pouch with the insoluble residue in g

By evaporating the xylene from the xylene solution the xylene hot soluble fraction was obtained. This fraction was dried at 60 °C under vacuum overnight prior to further analysis with other analytical tests like NMR spectroscopy.

**i) Flexural modulus**

The flexural modulus was determined according to ISO 178 method A (3-point bending test) on specimens having a dimension of 80 mm × 10 mm × 4 mm (length x width x thickness). Following the standard, a test speed of 2mm/min and a span length of 16x thickness was used. The testing temperature was 23±2° C. Injection moulding was carried out according to ISO 17855-2 for specimen preparation.

**j) FNCT** (full notch creep test)

FNCT time to failure was measured according to the full notch creep test method (FNCT) ISO 16770-2019 at 50° C with a notch depth of 1 mm and specimen dimensions 6 mm × 6 mm × 90 mm (4 mm × 4mm). The solvent used was 2 wt % Arkopal N100 in deionized water. The sample preparation was done following ISO 16670-2019 (compression moulding and annealing).
The test specimens were stressed in an aqueous solution at 6 MPa stress. For each sample, 3 to 4 specimens were tested. The average time to failure value of all the measurements were used to report the time to failure in hours.

**k) ESCR** (environmental stress crack resistance - Bottle test)

The 1L bottle samples have been produced on extrusion blow moulding line from the company Uniloy Milacron Germany GmbH. The extrusion blow moulding line consists of an extruder with a Ø 60 mm screw with an L/D ration of 24. The extruder temperature has been set at 170-200°C, the melt temperature is 190-210°C and has been recorded after 60 min of process stabilization. The extruder is followed by an extrusion hose head, which is equipped with an Ø 21-24 mm annular die and with a pin of Ø 15-18 mm to adjust a die gap of 0,5 - 4,0mm. The line has been run with a speed of 10-15 rpm. The bottle was produced with a thickness of 0,58 - 0,62 mm.
The Environmental Stress-Crack Resistance of the bottle samples is measured following the general guidelines of the standard ASTM D2561 - 17, on an internal pressure bottle test set-up manufactured by Hammerschmid Maschinenbau GmbH.
Each bottle is filled with 100 ml of the stress-cracking fluid. The fluid is a solution of 10% volume of IGEPAL® CA-630 on de-ionized water. The bottles are then heated to 60 ± 1°C, and the temperature throughout is kept and controlled during the entire test.
The machine applied the internal controlled pressure of 0.35±0.03 bar. Every minute the device controls the pressure of each bottle, and if the pressure drops 0.01 bar from the set-point, the bottle is re-pressurized. In the same control, the bottle is considered broken if the pressure drops below 0.05 bar from the set-point. The specimens are visually controlled five times per week, and the fluid in the bottle is shaken. At least eight bottles are tested per material, and the average time to failure is reported.

**Examples**

**a) Recycling process**

Several post-consumer plastic trash HDPE streams from separate plastic waste collection (civic amenity sites offering specific HDPE collections) were coarsely sorted as to polymer nature and as to color. Impurities were further separated by manual inspection.

a) whereby receiving respective streams of polyethylene material, a first stream being essentially transparent and a second stream being essentially of white color,
b) subjecting the streams separately to milling, washing in an aqueous solution with various detergents and subsequent drying, windsifting and screening yielding two pretreated streams;
c) subjecting the two pretreated streams (all; separately) to a further sorting for eliminating non-polyolefin and possibly colored parts;
d) extruding into pellets;
e) optionally aeration which is preferably carried out at a temperature in a 30 range of 100-130°C by preheating the post-consumer plastic material to such temperature using an air stream having a temperature of at least 100°C for at least 20 hours.

Aeration is usually necessary but may be skipped under specific circumstances.
Odor control and assessment is possible by a number of methods. An overview is provided inter alia by Demets, Ruben, et al. "Development and application of an analytical method to quantify odour removal in plastic waste recycling processes." Resources, Conservation and Recycling 161 (2020): 104907 being incorporated by reference 5 herewith.
According to said sorting process 4 natural polyethylene based blends (PE1, PE2, PE3 and PE4) and 3 white polyethylene based blends (PE5, PE6 and PE7) were obtained.

**b) Analysis of the blends**

All polyethylene based blends PE1 to PE7 were subjected to analysis of their composition and properties as listed in Table 1 below.

Table 1: Composition and properties of polyethylene based blends PE1 to PE7

| | PE1 | PE2 | PE3 | PE4 | PE5 | PE6 | PE7 |
|---|---|---|---|---|---|---|---|
| Feedstock type | Natural | Natural | Natural | Natural | White | White | White |
| **Properties:** | | | | | | | |
| Density [kg/m$^3$] | 958.1 | 957.8 | 959.5 | 959.4 | 968.5 | 968.5 | 962.7 |
| MFR$_2$ [g/10 min] | 0.32 | 0.49 | 0.51 | 0.46 | 0.34 | 0.32 | 0.48 |
| MFR$_5$ [g/10 min] | 1.38 | 2.2 | 2.16 | 2.02 | 1.51 | 1.42 | |
| MFR$_{21}$ [g/10 min] | 28.52 | 41.22 | 43.12 | 39.86 | 30.34 | 29.57 | 40.78 |
| FRR$_{21/2}$ | 89.1 | 84.1 | 84.5 | 86.7 | 89.2 | 92.4 | 85.0 |
| FRR$_{21/5}$ | 20.7 | 18.7 | 20.0 | 19.7 | 20.1 | 20.8 | |
| Eta$_{0.05}$ [Pa·s] | 39868 | 34336 | 34724 | 36076 | 39541 | 44145 | 33975 |
| Eta$_{300}$ [Pa·s] | 832 | 703 | 697 | 713 | 800 | 786 | 700 |
| PI [s$^{-1}$] | 2.21 | 2.27 | 2.36 | 2.39 | 2.31 | 2.55 | 2.36 |
| SHI$_{2.7/210}$ | 32.92 | 39.12 | 40.71 | 41.17 | 35.85 | 43.2 | 39.56 |
| $\omega_{COP}$ [rad/s] | 13.08 | 21.24 | 19.74 | 17.64 | 13.42 | 11.37 | 19.11 |

(continued)

| Properties: | | | | | | | |
|---|---|---|---|---|---|---|---|
| Flexural modulus [MPa] | 937 | 964 | 979 | 991 | 928 | 905 | 995 |
| FNCT time to failure [h] | 19.1 | 3.8 | 2.9 | 6.5 | 14.2 | 6.5 | 6.7 |
| Bottle ESCR time to failure [h] | 34.6 | 7.5 | 4.2 | | | 18.7 | 12.4 | |
| **Composition:** | | | | | | | |
| Chalk [wt%] | 0.1 | | n.m. | n.m. | n.m. | n.m. | n.m. |
| PA [wt%] | n.m. | | n.m. | n.m. | n.m. | n.m. | n.m. |
| PS [wt%] | n.m. | | n.m. | n.m. | n.m. | n.m. | n.m. |
| Talc [wt%] | n.m. | | n.m. | n.m. | n.m. | n.m. | n.m. |
| PVC [wt%] | n.m. | | n.m. | n.m. | n.m. | n.m. | n.m. |
| PET [wt%] | n.m. | | n.m. | n.m. | n.m. | n.m. | n.m. |
| EVA [wt%] | n.m. | | n.m. | n.m. | n.m. | n.m. | n.m. |
| iPP [wt%] | 1 | | 1 | n.m. | 1 | 1 | n.m. |
| C2 total [wt%] | 99.59 | 99.80 | 99.72 | 100 | 99.02 | 99.26 | 99.77 |
| C3 total (isolated C3) [wt%] | n.m. | n.m. | n.m. | n.m. | n.m. | n.m. | n.m. |
| C4 total [wt%] | 0.31 | n.m. | 0.09 | n.m. | 0.38 | 0.18 | n.m. |
| C6 total [wt%] | n.m. | 0.20 | 0.19 | n.m. | 0.32 | 0.26 | n.m. |
| C7 total [wt%] | n.m. | n.m. | n.m. | n.m. | n.m. | n.m. | n.m. |
| Continuous C3 [wt%] | 0.10 | 0 | 0 | 0 | 0.28 | 0.30 | 0.23 |
| PE C2 rich fraction [wt%] | 99.9 | 100 | 100 | 100 | 99.7 | 99.7 | 99.8 |
| OCS contaminations 100-299 $\mu$m [1/m$^2$] | n.m. | n.m. | n.m. | n.m. | n.m. | n.m. | 27.1 |
| OCS contaminations 300-2599 $\mu$m [1/m$^2$] | n.m. | n.m. | n.m. | n.m. | n.m. | n.m. | 37.9 |
| OCS contaminations 600-1000 $\mu$m [1/m$^2$] | n.m. | n.m. | n.m. | n.m. | n.m. | n.m. | 17.7 |
| OCS contaminations >1000 $\mu$m [1/m$^2$] | n.m. | n.m. | n.m. | n.m. | n.m. | n.m. | 8.5 |
| OCS gels 100-299 $\mu$m [1/m$^2$] | 973.5 | 823.2 | 971.8 | 2073.6 | 1620.9 | 2960.1 | 3806.6 |
| OCS gels 300-2599 $\mu$m [1/m$^2$] | 283.1 | 148.7 | 174.6 | 503.7 | 863.8 | 1916.9 | 1188.0 |
| OCS gels 600-1000 $\mu$m [1/m$^2$] | 22.0 | 25.0 | 20.9 | 81.2 | 33.6 | 116.7 | 155.0 |
| OCS gels >1000 $\mu$m [1/m$^2$] | 1.6 | 3.8 | 4.7 | 9.2 | 7.1 | 27.2 | 11.2 |
| Ash content [wt%] | 0.1 | 0.03 | 0.02 | 0.08 | 1.17 | 1.57 | 0.43 |
| Water content [ppm] | 138 | 17 | | | 118 | 92.4 | |
| XHU [wt%] | 0.02 | 0.02 | 0.01 | 0.01 | 0.42 | 0.52 | 0.13 |
| Al [ppm] | 24 | n.m. | n.m. | n.m. | 141 | 136 | |
| Ba [ppm] | n.m. | n.m. | n.m. | n.m. | n.m. | 139 | |
| Br [ppm] | n.m. | n.m. | n.m. | n.m. | n.m. | n.m. | |
| Ca [ppm] | 156 | 90 | 86 | 86 | 869 | 404 | |
| Cd [ppm] | n.m. | n.m. | n.m. | n.m. | n.m. | n.m. | |
| Cl [ppm] | 28 | 16 | n.m. | 21 | 29 | 64 | |

(continued)

| Composition: | | | | | | | |
|---|---|---|---|---|---|---|---|
| Cr [ppm] | 1 | 2 | 2 | 3 | n.m. | n.m. | |
| Cu [ppm] | n.m. | n.m. | n.m. | n.m. | n.m. | n.m. | |
| Fe [ppm] | 38 | 35 | n.m. | n.m. | 39 | n.m. | |
| K [ppm] | n.m. | n.m. | n.m. | n.m. | 20 | n.m. | |
| Mg [ppm] | n.m. | n.m. | n.m. | n.m. | n.m. | 55 | |
| Na [ppm] | 27 | n.m. | n.m. | 27 | 25 | 29 | |
| Ni [ppm] | n.m. | n.m. | n.m. | n.m. | n.m. | n.m. | |
| P [ppm] | 89 | 81 | 91 | 72 | 93 | 101 | |
| Pb [ppm] | n.m. | n.m. | n.m. | n.m. | n.m. | n.m. | |
| S [ppm] | n.m. | n.m. | n.m. | n.m. | 48 | 52 | |
| Sb [ppm] | n.m. | n.m. | n.m. | n.m. | n.m. | n.m. | |
| Si [ppm] | 64 | 104 | 132 | 131 | 167 | 225 | |
| Sr [ppm] | n.m. | n.m. | n.m. | n.m. | n.m. | n.m. | |
| Ti [ppm] | 325 | 33 | 30 | 70 | 8001 | 7658 | |
| Zn [ppm] | 4 | n.m. | n.m. | n.m. | 68 | 81 | |
| n.m.: not measurable | | | | | | | |

### c) Establishment of correlations

The polyethylene based blends PI to PE7 were used as first amount of polyethylene based blends.

The accordant values for etao.os, $SHI_{2.7/210}$ and FNCT time to failure were used for establishing correlation (1):

$$\text{Pred. FNCT time to failure (h)} = 38.962 + 6.806E\text{-}4 \cdot \text{eta}_{0.05} \, (\text{Pa·s}) - 1.438 \cdot SHI_{2.7/210} \quad (1)$$

The accordant values for etao.os, PI and FNCT time to failure were used for establishing correlation (2):

$$\text{Pred. FNCT time to failure (h)} = 69.915 + 0.0013 \cdot \text{eta}_{0.05} \, (\text{Pa·s}) - 46.949 \cdot PI \, (1/\text{Pa}) \quad (2)$$

The accordant values for $MFR_2$, $FRR_{21/2}$ and FNCT time to failure were used for establishing correlation (3):

$$\text{Pred. FNCT time to failure (h)} = 335.812 - 153.036 \cdot MFR_2 \, (\text{g/10 min}) - 3.018 \cdot FRR_{21/2} \quad (3)$$

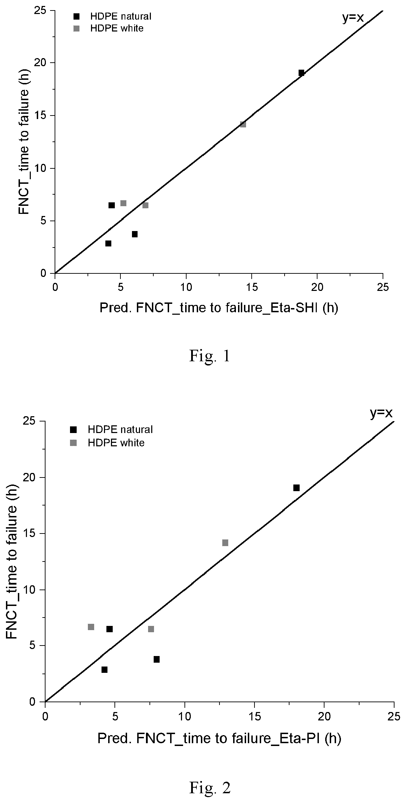

Figure 1 shows the graph of the measured FNCT time to failure function of the predicted FNCT time to failure calculated by means of correlation (1) ($\text{eta}_{0.05}$, $SHI_{2.7/210}$ and FNCT time to failure).

Figure 2 shows the graph of the measured FNCT time to failure function of the predicted FNCT time to failure calculated by means of correlation (2) ($\text{eta}_{0.05}$, PI and FNCT time to failure).

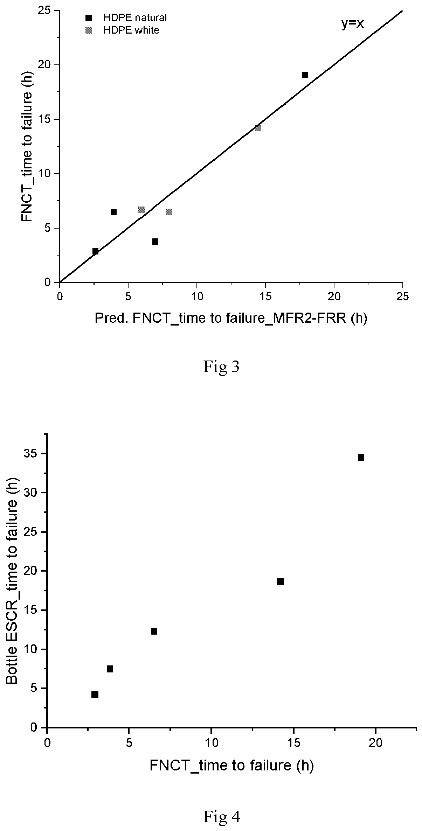

Figure 3 shows the graph of the measured FNCT time to failure function of the predicted FNCT time to failure calculated by means of correlation (3) ($MFR_2$, $FRR_{21/2}$ and FNCT time to failure).

Figure 4 shows that the bottles made of the above described PE compositions have ESCR results correlated with the FNCT time to failures of their notched specimens. Hence, by measuring one first property related to the broadness of the molecular weight distribution curve and one second property related to the molecular weight, it is possible to estimate the long term properties of the converted articles such as a bottle.

**Claims**

1. A method of blending polyethylene based blends comprising the steps of

   • Providing a first amount of polyethylene based blends;
   • Measuring properties of said first amount of polyethylene based blends, wherein at least one first property is related to the broadness of the molecular weight distribution curve of each of the first amount of polyethylene based blends, at least one second property is related to the molecular weight of each of the first amount of polyethylene based blends and one property related to the target property of a certain application for which the blended polyethylene based blends are intended to be used;
   • Establishing a correlation between the at least one first property, the at least one second property and the one property related to the target property;
   • Providing a second amount of polyethylene based blends;
   • Measuring the same at least one first property and at least one second property, that were measured of the first amount of polyethylene based blends, of each of the second amount of polyethylene based blends;
   • Using the correlation between the first property, the second property and the property related to the target property established for the first amount of polyethylene based blends for predicting the one property related to the target property for each of the second amount of polyethylene based blends;
   • Setting a threshold for the one property related to the target property for polyethylene based blends to be selected for blending;
   • Selecting the blends of the second amount of polyethylene based blends, at least one of which meet the set threshold of the predicted property related to the target property;
   • Blending said selected blends.

2. The method according to claim 1, wherein the at least one first property related to the broadness of the molecular weight distribution curve is selected from

   1) parameter(s) determined from dynamic shear measurements, such as shear thinning index $SHI_{2.7/210}$, polydispersity index PI,
   2) parameter(s) determined according to ISO 1133, such as flow rate ratio $FRR_{21/2}$ ($MFR_{21}/MFR_2$) that is calculated with the melt flow rates at a temperature of 190°C and loads of 21.6 kg and 2.16 kg,
   3) parameter(s) determined from GPC measurement, such as Mw/Mn,
   4) and combinations thereof.

3. The method according to claim 1 or 2, wherein the at least one second property related to the molecular weight is selected from

   1) parameter(s) determined from dynamic shear measurements, such as $eta_{0.05}$ (viscosity at 0.05 rad/s),
   2) parameter(s) determined according to ISO 1133, such as $MFR_2$, determined at a temperature of 190°C and loads of 2.16 kg,
   3) parameter(s) determined from GPC measurement, such as weight average molecular weight Mw,
   4) and combinations thereof.

4. The method according to any one of the preceding claims, wherein the target property is slow crack growth resistance.

5. The method according to any one of the preceding claims, wherein the property related to the target property is selected from full notch creep test (FNCT), determined according to ISO 16770 or environmental stress crack resistance (ESCR) of bottle according to ASTM D2561.

6. The method according to any one of the preceding claims, wherein each blend of the first amount of polyethylene based blends has one or more or all of the following properties:

   • a melt flow rate $MFR_2$ of from 0.01 to 2.5 g/10 min, more preferably from 0.05 to 2.0 g/10 min, still more preferably from 0.1 to 1.5 g/10 min, determined according to ISO 1133 at 190°C and 2.16 kg;
   • a melt flow rate $MFR_5$ of from 0.1 to 5.0 g/10 min, more preferably from 0.5 to 4.0 g/10 min, still more preferably from 1.0 to 3.0 g/10 min, determined according to ISO 1133 at 190°C and 5 kg;
   • a melt flow rate $MFR_{21}$ of from 20 to 55 g/10 min, more preferably from 22 to 50 g/10 min, still more preferably from 25 to 45 g/10 min, determined according to ISO 1133 at 190°C and 21.6 kg;

• a flow rate ratio $FRR_{21/2}$, being the ratio of $MFR_{21}$ to $MFR_2$, of from 70 to 110, more preferably from 75 to 100, still more preferably from 80 to 95;

• a flow rate ratio $FRR_{21/5}$, being the ratio of $MFR_{21}$ to $MFR_5$, of from 10 to 30, more preferably from 12 to 27, more preferably from 15 to 25;

• a polydispersity index PI of from 1.5 to 3.5 $s^{-1}$, more preferably from 2.2 to 3.2 $s^{-1}$, still more preferably from 2.0 to 3.0 $s^{-1}$, determined from dynamic shear measurements;

• a shear thinning index $SHI_{2.7/210}$ of from 25 to 50, more preferably from 27 to 47, still more preferably 30 to 45, determined from dynamic shear measurements;

• a complex viscosity at a frequency of 0.05 rad/s $eta_{0.05}$ of from 25,000 to 55,000 Pa·s, more preferably from 27,500 to 52,500 Pa·s, still more preferably from 30,000 to 50,000 Pa·s, determined from dynamic shear measurements;

• a complex viscosity at a frequency of 300 rad/s $eta_{300}$ of from 500 to 1000 Pa·s, more preferably from 600 to 900 Pa·s, still more preferably from 650 to 850 Pa·s, determined from dynamic shear measurements;

• a density of from 950 to 975 $kg/m^3$, preferably from 952 to 972 $kg/m^3$, more preferably from 955 to 970 $kg/m^3$, determined according to ISO 1183;

• an amount of contaminants, preferably selected from chalk, talc, titanium dioxide, ash, polyamide, polystyrene and polypropylene, not more than 4.0 wt%, preferably from 0 to 3.0 wt%, more preferably from 0 to 2.5 wt%, based on the total weight of each blend, determined by FTIR;

• an ash content of not more than 3.0 wt%, preferably from 0 to 2.5 wt%, more preferably from 0 to 2.0 wt%, based on the total weight of each blend, determined by TGA;

• a titanium content of not more than 10,000 ppm, preferably from 0 to 9,000 ppm, more preferably from 0 to 8,500 ppm, based on the total weight of each blend, determined by XRF;

• an amount of polyethylene based polymer fraction of at least 97.0 wt%, preferably from 97.0 to 100 wt%, more preferably from 98.0 to 100 wt%, based on the total weight of each blend, determined by NMR;

• an amount of ethylene monomer units in the polyethylene based polymer fraction of from 98.0 to 100 wt%, preferably from 98.5 to 100 wt%, more preferably from 99.0 to 100 wt%, based on the total weight of the polyethylene based polymer fraction, determined by NMR;

• an amount of alpha-olefin comonomer units in the polyethylene based polymer fraction of from 0 to 2.0 wt%, preferably from 0 to 1.5 wt%, more preferably from.0 to 1.0 wt%, based on the total weight of the polyethylene based polymer fraction, determined by NMR.

**7.** The method according to any one of the preceding claims, wherein each blend of the first amount of polyethylene based blends and/or each blend of the second amount of polyethylene based blends is a natural blend or a white blend.

**8.** The method according to any one of the preceding claims, wherein each blend of the second amount of polyethylene based blends has one or more or all of following properties:

• a melt flow rate $MFR_2$ of from 0.01 to 2.5 g/10 min, more preferably from 0.05 to 2.0 g/10 min, still more preferably from 0.1 to 1.5 g/10 min, determined according to ISO 1133 at 190°C and 2.16 kg;

• a melt flow rate $MFR_5$ of from 0.1 to 5.0 g/10 min, more preferably from 0.5 to 4.0 g/10 min, still more preferably from 1.0 to 3.0 g/10 min, determined according to ISO 1133 at 190°C and 5 kg;

• a melt flow rate $MFR_{21}$ of from 20 to 55 g/10 min, more preferably from 22 to 50 g/10 min, still more preferably from 25 to 45 g/10 min, determined according to ISO 1133 at 190°C and 21.6 kg;

• a flow rate ratio $FRR_{21/2}$, being the ratio of $MFR_{21}$ to $MFR_2$, of from 70 to 110, more preferably from 75 to 100, still more preferably from 80 to 95;

• a flow rate ratio $FRR_{21/5}$, being the ratio of $MFR_{21}$ to $MFR_5$, of from 10 to 30, more preferably from 12 to 27, more preferably from 15 to 25;

• a polydispersity index PI of from 1.5 to 3.5 $s^{-1}$, more preferably from 2.2 to 3.2 $s^{-1}$, still more preferably from 2.0 to 3.0 $s^{-1}$, determined from dynamic shear measurements;

• a shear thinning index $SHI_{2.7/210}$ of from 25 to 50, more preferably from 27 to 47, still more preferably 30 to 45, determined from dynamic shear measurements;

• a complex viscosity at a frequency of 0.05 rad/s $eta_{0.05}$ of from 25,000 to 55,000 Pa·s, more preferably from 27,500 to 52,500 Pa·s, still more preferably from 30,000 to 50,000 Pa·s, determined from dynamic shear measurements;

• a complex viscosity at a frequency of 300 rad/s $eta_{300}$ of from 500 to 1000 Pa·s, more preferably from 600 to 900 Pa·s, still more preferably from 650 to 850 Pa·s, determined from dynamic shear measurements;

• a density of from 950 to 975 $kg/m^3$, preferably from 952 to 972 $kg/m^3$, more preferably from 955 to 970 $kg/m^3$, determined according to ISO 1183;

• an amount of contaminants, preferably selected from chalk, talc, titanium dioxide, ash, polyamide, polystyrene and polypropylene not more than 4.0 wt%, preferably from 0 to 3.0 wt%, more preferably from 0 to 2.5 wt%, based on the total weight of each blend, determined by FTIR;

• an ash content of not more than 3.0 wt%, preferably from 0 to 2.5 wt%, more preferably from 0 to 2.0 wt%, based on the total weight of each blend, determined by TGA;

• a titanium content of not more than 10,000 ppm, preferably from 0 to 9,000 ppm, more preferably from 0 to 8,500 ppm, based on the total weight of each blend, determined by XRF;

• an amount of polyethylene based polymer fraction of at least 97.0 wt%, preferably from 97.0 to 100 wt%, more preferably from 98.0 to 100 wt%, based on the total weight of each blend, determined by NMR;

• an amount of ethylene monomer units in the polyethylene based polymer fraction of from 98.0 to 100 wt%, preferably from 98.5 to 100 wt%, more preferably from 99.0 to 100 wt%, based on the total weight of the polyethylene based polymer fraction, determined by NMR;

• an amount of alpha-olefin comonomer units in the polyethylene based polymer fraction of from 0 to 2.0 wt%, preferably from 0 to 1.5 wt%, more preferably from.0 to 1.0 wt%, based on the total weight of the polyethylene based polymer fraction, determined by NMR.

9. The method according to any one of the preceding claims, wherein the first amount of polyethylene based blends and/or the second amount of polyethylene based blends comprise one or more recycled polyethylene based blends, which are obtained from sorting processes for sorting post-consumer waste and/or post-industrial waste.

10. The method according to any one of the preceding claims, wherein the at least one first property, the at least one second property and the one property related to the target property are correlated by multiple linear regression.

11. The method according to any one of the preceding claims, wherein the at least one first property is shear thinning index $SHI_{2.7/210}$, the at least one second property is viscosity at 0.05 rad/s $eta_{0.05}$ and the property related to the target property is full notch creep test FNCT and the correlation is

$$\text{Pred. FNCT time to failure (h)} = a + b \cdot eta_{0.05} \, (\text{Pa·s}) - c \cdot SHI_{2.7/210} \qquad (1)$$

with

Pred. FNCT time to failure being the FNCT time to failure predicted by correlation (1);
a being in the range of from 30.000 to 48.000, preferably from 33.000 to 45.000;
b being in the range of from 5.000E-4 to 7.500E-4, preferably from 5.500E-4 to 7.000E-4; and
c being in the range of from 1.300 to 1.600, preferably from 1.400 to 1.480.

12. The method according to any one of claims 1 to 10, wherein the at least one first property is polydispersity index PI, the at least one second property is viscosity at 0.05 rad/s etao.os and the property related to the target property is full notch creep test FNCT and the correlation is

$$\text{Pred. FNCT time to failure (h)} = d + e \cdot eta_{0.05} \, (\text{Pa·s}) - f \cdot PI \, (1/\text{Pa}) \qquad (2)$$

with

Pred. FNCT time to failure being the FNCT time to failure predicted by correlation (2);
d being in the range of from 65.000 to 90.000, preferably from 67.500 to 88.000;
e being in the range of from 0.00050 to 0.00200, preferably from 0.00100 to 0.00150; and
f being in the range of from 40.000 to 55.000, preferably from 45.000 to 52.000.

13. The method according to any one of claims 1 to 10, wherein the at least one first property is flow rate ratio $FRR_{21/2}$, the at least one second property is melt flow rate $MFR_2$ and the property related to the target property is full notch creep test FNCT and the correlation is

$$\text{Pred. FNCT time to failure (h)} = g + h \cdot MFR_2 \, (\text{g/10 min}) - i \cdot FRR_{21/2} \qquad (3)$$

with

> Pred. FNCT time to failure being the FNCT time of failure predicted by correlation (3);
> g being in the range of from 265.000 to 400.000, preferably from 330.000 to 390.000;
> h being in the range of from 140.000 to 165.000, preferably from 150.000 to 162.500; and
> i being in the range of from 2.500 to 4.000, preferably from 2.750 to 3.750.

14. Polyethylene based blends blended by the method according to any one of claims 1 to 13 having a time to failure of at least 5 hours in full notch creep test (FNCT), determined according to ISO 16770 or a time to failure of at least 10 hours in environmental stress crack resistance (ESCR) test of bottle according to ASTM D2561.

15. Article comprising polyethylene based blends according to claim 14.

Fig. 1

Fig. 2

Fig 3

Fig 4

Europäisches
Patentamt

European
Patent Office

Office européen
des brevets

**EUROPEAN SEARCH REPORT**

Application Number

**EP 22 19 3846**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/171492 A1 (STARITA JOSEPH M [US]) 11 September 2003 (2003-09-11) | 1-15 | INV. C08J3/00 G01N33/44 C08J11/06 |
| Y | * paragraphs [0002] – [0005], [0030], [0031], [0033], [0039] – [0041], [0054] * <br> * paragraphs [0059] – [0076], [0091], [0092] * <br> * figures 11, 12 * <br> * claims 1-20 * | 1-13 | |
| X | US 4 547 551 A (BAILEY FAY W [US] ET AL) 15 October 1985 (1985-10-15) * tables VI, VIII, IX, XI-XIII, XV, XVI, XVIII * | 14,15 | |
| X | US 2017/107364 A1 (TROLEZ YVES [BE] ET AL) 20 April 2017 (2017-04-20) * table 4 * * claim 1 * | 14,15 | |
| Y | US 2010/319440 A1 (DESLAURIERS PAUL J [US] ET AL) 23 December 2010 (2010-12-23) * figures 1-26 * * claims 1, 4, 7, 14, 16, 18-20 * | 1-13 | TECHNICAL FIELDS SEARCHED (IPC) C08J G01N |
| Y | US 2019/064051 A1 (HONG YOON KI [KR] ET AL) 28 February 2019 (2019-02-28) * figures 1, 2 * * claims 1, 2 * | 1-13 | |
| Y | US 2019/086308 A1 (LEE HYUN SUP [KR] ET AL) 21 March 2019 (2019-03-21) * claim 1 * | 1-13 | |

-/--

The present search report has been drawn up for all claims

| Place of search | Date of completion of the search | Examiner |
|---|---|---|
| Munich | 7 February 2023 | Vandoolaeghe, P |

EPO FORM 1503 03.82 (P04C01)

page 1 of 2

# EUROPEAN SEARCH REPORT

Application Number

EP 22 19 3846

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Amirpouyan Sardashti: "Methodologies for Obtaining Reliable Indicators for the Environmental Stress Cracking Resistance of Polyethylene", A Thesis for the Degree of Doctor of Philosophy, 13 February 2014 (2014-02-13), pages 1-263, XP055586856, Retrieved from the Internet: URL:https://uwspace.uwaterloo.ca/bitstream /handle/10012/8255/Sardashti_Amirpouyan.pd f?sequence=5&isAllowed=y [retrieved on 2019-05-08] * the whole document * | 1-15 | |

TECHNICAL FIELDS
SEARCHED      (IPC)

The present search report has been drawn up for all claims

| Place of search | Date of completion of the search | Examiner |
|---|---|---|
| Munich | 7 February 2023 | Vandoolaeghe, P |

EPO FORM 1503 03.82 (P04C01)

## ANNEX TO THE EUROPEAN SEARCH REPORT
## ON EUROPEAN PATENT APPLICATION NO.

**EP 22 19 3846**

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2023

| Patent document cited in search report | | | Publication date | Patent family member(s) | | | Publication date |
|---|---|---|---|---|---|---|---|
| US 2003171492 | A1 | | 11-09-2003 | AU | 2003218209 | A1 | 02-02-2004 |
| | | | | BR | 0305064 | A | 21-09-2004 |
| | | | | CA | 2455946 | A1 | 22-01-2004 |
| | | | | CN | 1556835 | A | 22-12-2004 |
| | | | | EP | 1448703 | A1 | 25-08-2004 |
| | | | | EP | 1676883 | A2 | 05-07-2006 |
| | | | | MX | PA04001345 | A | 22-11-2004 |
| | | | | US | 2003171492 | A1 | 11-09-2003 |
| | | | | WO | 2004007610 | A1 | 22-01-2004 |
| US 4547551 | A | | 15-10-1985 | NONE | | | |
| US 2017107364 | A1 | | 20-04-2017 | DK | 3074464 | T3 | 06-06-2017 |
| | | | | EP | 3074464 | A1 | 05-10-2016 |
| | | | | ES | 2632482 | T3 | 13-09-2017 |
| | | | | HR | P20170727 | T1 | 22-09-2017 |
| | | | | PL | 3074464 | T3 | 29-09-2017 |
| | | | | PT | 3074464 | T | 03-07-2017 |
| | | | | SI | 3074464 | T1 | 31-08-2017 |
| | | | | US | 2017107364 | A1 | 20-04-2017 |
| | | | | WO | 2016005265 | A1 | 14-01-2016 |
| US 2010319440 | A1 | | 23-12-2010 | AU | 2007265111 | A1 | 03-01-2008 |
| | | | | BR | PI0712982 | A2 | 10-04-2012 |
| | | | | CA | 2655850 | A1 | 03-01-2008 |
| | | | | CN | 101512335 | A | 19-08-2009 |
| | | | | EP | 2038649 | A2 | 25-03-2009 |
| | | | | EP | 2469277 | A1 | 27-06-2012 |
| | | | | ES | 2536766 | T3 | 28-05-2015 |
| | | | | ES | 2661580 | T3 | 02-04-2018 |
| | | | | RU | 2009102541 | A | 10-08-2010 |
| | | | | US | 2007298508 | A1 | 27-12-2007 |
| | | | | US | 2010319440 | A1 | 23-12-2010 |
| | | | | WO | 2008002969 | A2 | 03-01-2008 |
| US 2019064051 | A1 | | 28-02-2019 | CN | 108351332 | A | 31-07-2018 |
| | | | | EP | 3355053 | A1 | 01-08-2018 |
| | | | | KR | 20180058575 | A | 01-06-2018 |
| | | | | US | 2019064051 | A1 | 28-02-2019 |
| | | | | WO | 2018097477 | A1 | 31-05-2018 |
| US 2019086308 | A1 | | 21-03-2019 | CN | 108885224 | A | 23-11-2018 |
| | | | | EP | 3517976 | A1 | 31-07-2019 |
| | | | | JP | 6685544 | B2 | 22-04-2020 |
| | | | | JP | 2019529950 | A | 17-10-2019 |
| | | | | KR | 20180058574 | A | 01-06-2018 |

EPO FORM P0459

For more details about this annex : see Official Journal of the European Patent Office, No. 12/82

**page 1 of 2**

## ANNEX TO THE EUROPEAN SEARCH REPORT
## ON EUROPEAN PATENT APPLICATION NO.

EP 22 19 3846

This annex lists the patent family members relating to the patent documents cited in  the above-mentioned European search report.
The members are as contained in the European Patent Office EDP file on
The European Patent Office is in no way liable for these particulars which are merely given for the purpose of information.

07-02-2023

| Patent document cited in search report | Publication date | Patent family member(s) | | Publication date |
|---|---|---|---|---|
| | | US | 2019086308 A1 | 21-03-2019 |
| | | WO | 2018097508 A1 | 31-05-2018 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2022064702 W

**Non-patent literature cited in the description**

- **DR. FRANK WELLE.** Develop a food grade HDPE recycling process. *The Waste & Resources Action Programme,* 04 June 2005, https://www.research-gate.net/profile/Frank-Welle-2/publication/284158562_Develop_a_food_grade_HDPE_recycling_process/links/564ca7df0 8ae7ac727e20707/Develop-a-food-grade-HDPE-recycling-process.pdf?origin=publication_detail
- *CHEMICAL ABSTRACTS,* 128-37-0
- **ZHOU, Z. ; KUEMMERLE, R. ; QIU, X. ; REDWINE, D. ; CONG, R. ; TAHA, A. ; BAUGH, D. ; WINNIFORD, B.** *J. Mag. Reson.,* 2007, vol. 187, 225
- **BUSICO, V. ; CARBONNIERE, P. ; CIPULLO, R. ; PELLECCHIA, R. ; SEVERN, J. ; TALARICO, G.** *Macromol. Rapid Commun.,* 2007, vol. 28, 1128
- **SINGH, G. ; KOTHARI, A. ; GUPTA, V.** *Polymer Testing,* 2009, vol. 28 (5), 475
- **J. RANDALL.** *Macromol. Sci., Rev. Macromol. Chem. Phys.,* 1989, vol. C29, 201
- **A. J. BRANDOLINI ; D. D. HILLS.** NMR Spectra of Polymers and Polymer Additives. Marcel Dekker Inc, 2000

- **SIGNORET.** Alterations of plastic spectra in MIR and the potential impacts on identification towards recycling. *Resources, conservation and Recycling journal,* 2020, vol. 161
- **HEINO, E.L. ; LEHTINEN, A. ; TANNER J. ; SEPPÄLÄ, J.** Rheological characterization of polyethylene fractions. *Neste Oy, Porvoo, Finland, Theor. Appl. Rheol., Proc. Int. Congr. Rheol,* 1992, vol. 1, 360-362
- **HEINO, E.L.** The influence of molecular structure on some rheological properties of polyethylene. *Borealis Polymers Oy, Porvoo, Finland, Annual Transactions of the Nordic Rheology Society,* 1995
- Definition of terms relating to the non-ultimate mechanical properties of polymers. *Pure & Appl. Chem.,* 1998, vol. 70 (3), 701-754
- **DEMETS, RUBEN et al.** Development and application of an analytical method to quantify odour removal in plastic waste recycling processes. *Resources, Conservation and Recycling,* 2020, vol. 161, 104907